# EUROPEAN PATENT APPLICATION

(11) **EP 1 970 044 A1**
(43) Date of publication of application: **17.09.2008**
(21) Application number: 07104209.7
(22) Date of filing: 15.03.2007
(51) Int. Cl.: A61K 8/25, A61K 8/60, A61K 8/73, A61Q 5/06

(54) **Solid form-stable gels comprising saccharide**

(71) Applicant: Wella Aktiengesellschaft, 64274 Darmstadt (DE)
(72) Inventor: Loifenfeld, Martina, 60316, Frankfurt (DE); Stein, Bernd, 63768, Hösbach (DE)

(57) **Abstract**

The present invention provides a hair treatment composition in the form of a solid, form-stable gel for hair treatment comprising at least one saccharide, at least one gel-forming ingredient, and an aqueous or aqueous-alcoholic base, wherein the ratio of the at least one saccharide to the at least one gel-forming ingredient within a certain range.

## Description

### FIELD OF THE INVENTION

The subject matter of the present invention includes hair treatment compositions in the form of solid, form-stable gels comprising at least one saccharide and at least one gel-forming ingredient.

### BACKGROUND OF THE INVENTION

Hair treatment compositions, among others, are used in the form a thickened preparations, e.g. gels, in order to fix and hold human hair or to stabilize a prepared hairdo or hairstyle. This type of product has a highly viscous, but still fluid, consistency. These gels are usually packaged in plastic tubes, from which they are forced out in the form of strands when they are used. They usually contain a gel forming ingredient, such as a polyacrylate, and a hair-fixing polymer, for example polyvinylpyrrolidone. The cosmetic hair-fixing polymers usually used for this purpose in conventional aqueous or aqueous-alcoholic gels have good fixing properties. They hold and fix the hair during application and stabilize an established hairdo or hairstyle.

Other known thickened preparations known for hair treatments are hair styling wax compositions. Compared to hair styling gels, styling waxes are typically much thicker and are usually provided in cups or other vessels. They find application particularly in putting short to medium length hair in a fashionable hairstyle and impart hold and luster. They also stabilize, condition and fix the hairstyle. Also a hair-do may be shaped and provided with texture with a hair wax. Conventional hair waxes are typically used as follows: The product is removed with the fingers. The wax is distributed on the surface of the hand and then melted or at least considerably softened by the heat of the hand. It is possible to work the otherwise too hard wax into the hair because of this softening or melting. The wax is worked into the hair in a softened or more or less liquid state. Then it cools and again reaches its original consistency. It hardens and the hairdo obtained has stability and hold and frequently a slightly wet look. Conventional hair wax products, as they are currently marketed, are usually based on hydrophobic, non-silicone containing waxes, fats and oils. They contain a large amount of hydrophobic materials, such as plant or animal waxes, fatty acid esters, fatty alcohols, etc. The main ingredients and primary effective ingredients are hydrophobic waxes.

Hair waxes are commonly opaque compositions, which often have a yellowish colour. This opaque appearance is disliked by a number of consumers, who -while liking the solid consistency of the wax- prefer a transparent, light appearance typical for gels. Also, some consumers do not like the waxy or oily feeling of the wax and would prefer the light, fresh feeling of a gel. Moreover, a wax typically provides the hair with a shiny wet look due to the wax, fat and oil ingredients. If consumers prefer a matt finish for their hair-do, this is difficult if not impossible to achieve with a wax.

Thus, it is on object of the present invention, to provide a hair styling gel with a solid consistency. The hair styling gel should preferably be able to provide a matt finish to the hair. Also, the hair styling gel should have a fresh, light consistency and should have a smooth touch.

### SUMMARY OF THE INVENTION

It has now been found that the objects of the present invention are attained by a hair treatment composition in the form of a solid, form-stable gel for hair treatment comprising
- at least one saccharide, and
- at least one gel-forming ingredient, and
- an aqueous or aqueous-alcoholic base
wherein the ratio of the at least one saccharide to the at least one gel-forming ingredient is between 1.5 : 1 and 5.5 : 1.

The words "solid, form-stable", in the sense of the present invention, mean that the gels according to the invention have wherein the gel has an Index of Viscosity at 20°C of between - 500 g s and -1500 g s. The Index of Viscosity is determined by Texture Analysis as described below.

### DETAILED DESCRIPTION OF THE INVENTION

### Definitions

Thixotropy: The property of a gel of becoming fluid when stirred or shaken and returning to the semisolid state upon standing.

In the present invention, the term "thixotropy" refers to gel-forming ingredients, which, when dissolved in aqueous or aqueous-alcoholic base -without further ingredients like the saccharide being present in the solution- show a thixotropic behavior.

Saccharide: Any of a series of compounds of carbon, hydrogen, and oxygen in which the atoms of the latter two elements are in the ratio of 2:1. Preferred sacchareides according to the present invention have the formula (C₆H₁₀O₅)_{n.} Especially preferred saccharides according to the present inventions are those having from 1 to 10 monosaccharide units covalently linked together, even more preferred saccharides are sugars. Sugars according to the present invention are mono-, di-or trisaccharides.

"Hair-fixing polymers" are understood to be those polymers, which, when used in an amount of from 0.01 to 5% in an aqueous, alcoholic or aqueous-alcoholic solution, are in a position to deposit a polymer film on the hair and to fix the hair.

### Saccharides

The composition according to the invention comprises at least one saccharide in order to improve the clarity and transparency of the composition as well as to provide the hair-fixing ability to the gel.

Monosaccharides and oligosaccharides having up to 10 monosaccharide units covalently linked together are preferred. With polysaccharides such as starch or cellulose it is more difficult to obtain a homogeneous gel. Most preferred saccharides according to the present inventon are are sugars. Sugars according to the present invention are mono-, di- or trisaccharides.

Suitable sugars, for example, include monosaccharides and disaccharides, such as glucose, galactose, fructose, maltose, lactose, saccharose or sucrose.

Moreover, preferred saccharides of the present invention only comprise carbon atoms, hydrogen atoms and oxygen atoms, more preferably they have the general formula CₙH₂ₙOₙ. and even more preferably they are either pentose or hexose monosaccharides or oligosaccarides made of pentose or hexose units or mixtures of pentose and hexose units.

Typically from 10% to 35% by weight, more preferably from 12% to 30% by weight, and even more preferably from 15% to 30% by weight of sugar is used in the composition according to the invention. If amounts of below 10% by weight are used, the gel may not be solid and form-stable enough. Also, the gel may not be able to provide the desired hold and definition to the hair-do. Amounts of above 35% by weight may lead to a gel that is too firm and compact and which can thus not be satisfactorily worked into the hair any more.

### Gel-forming ingredient

It has surprisingly been found that if the saccharide is used together with at least one gel-forming ingredient, a solid, form-stable gel with a pleasant, smooth texture is obtained if the ratio between these two types of ingredients is set within a certain range.

Suitable gel-formers or gel-forming ingredients (B) are, for example, Carbomers (acrylic acid polymers), especially those of the type Acrisint® 400, Xanthan gum or hydroxyalkylcellulose compounds, such as hydroxyethyl or hydroxypropylcellulose.

Preferred gel-forming ingredients according to the present invention are thixotropic gel-forming ingredients, as it has been surprisingly found that thixotropic gel-forming ingredients interact very well with the saccharides to provide the overall viscosity, consistency and rheology desired for gels of the present invention.

Suitable thixotropic gel-forming ingredients according to the present invention are silicates such as bentonites and kaoline, alginic acid, carrageenan, carbomers (acrylic acid polymers).

Preferred thixotropic gel-formers according to the present invention are carrageenan, e.g. a carrageenan sold under the trade name Genugel^{®} from CP Kelco, Atlanta, USA (especially Genugel^{®} type X-901-02) and silicates, e.g. a sodium magnesium silicate sold under the trade name Laponite^{®} from Solvay Alkali GmbH, Germany (especially Laponite^{®} XLG).

Typical concentrations of the thixotropic gel-forming ingredient are from 3.0% by weight to 10% by weight, preferably from 4% by weight to 8% by weight, even more preferably from 5.0% by weight to 7% by weight.

If amounts of below 3% by weight are used, the viscosity of the gel may be too low whereas with amounts of above 10% by weight the viscosity may be too high and it may become difficult to work the gel into the hair easily.

### Ratio of saccharides to thixotropic gel-forming ingredients

It has been found that a solid, form-stable gel can be formed if the ratio of saccharide to thixotropic gel-forming ingredient is between 1.5 : 1 and 5.5 : 1, preferably between 1.8 : 1 and 5 : 1, even more preferably between 2.0 : 1 and 5 : 1 and most preferably between 2.3 : 1 and 5:1. If other rations are used, the resulting gel is not homogeneous and tends to show a greasy consistency.

### Aqueous or Aqueous-Alcoholic Base

The gel according to the invention further comprises an aqueous base or aqueous-alcoholic base. The base can be either a purely aqueous medium or an aqueous-alcoholic medium with preferably up to 40 percent by weight alcohol. Lower univalent or multivalent alcohols suitable for cosmetic purposes and having from one to five carbon atoms, such as, e.g., ethanol, isopropanol, ethylene glycol, glycerol and propylene glycols, especially 1,2-propylene glycol, may be used as the alcohol. Typical water content is from 55 to 95 percent by weight and preferably from 65 to 80 percent by weight. Typical alcohol content is from 0 to 30 percent by weight and preferably from 1 to 25 percent by weight.

### Emulsifiers

In a preferred embodiment of the invention, the gel additionally comprises emulsifiers, preferably from the classes of anionic, cationic, amphoteric or nonionogenic surface-active substances, such as fatty alcohol sulfates, alkylbenzene sulfonates, alkyltrimethylammonium salts, moisturizers, alkyl betaines, ethoxylated fatty alcohols, ethoxylated nonylphenols, fatty acid alkanol amides, ethoxylated fatty acid esters. In the present invention the emulsifiers are preferably applied in an amount of from 1.0% by weight to 10.0% by weight, more preferably in an amount of 3.0% by weight to 8% by weight. If higher amounts of emulsifiers are used, the gel may become too slippery.

### Hair-Fixing Polymers

The gels of the present invention preferably comprise below 0.5% by weight of film-forming hair-fixing polymers, more preferably below 0.1% by weight. The gels of the present invention can also be free of any film-forming hair-fixing polymers, i.e. the gels do not comprise any film-forming hair-fixing polymers at all.

That is because in the gels of the present invention, the hair is mainly fixed by the saccharides comprised in the composition. A certain hair fixing action is also provided by the thixotropic gel-forming ingredient.

However, if the gel of the present invention comprises a low amount of a hair-fixing polymer, this hair-fixing polymer can be non-ionic, anionic, zwitterionic or amphoteric. Polymers, which do not contain cationic groups, i.e. anionic, nonionic and amphoteric polymers, are particularly preferred. Synthetic polymers are understood to be those polymers, which have a purely synthetic, i.e. not natural origin, especially those, which are made by radical polymerization from ethylenic unsaturated monomers or by polycondensation. Those polymers are particularly preferred, which have sufficient solubility or dispersibility in water, alcohol or alcohol/water mixtures, in order to be present in dissolved or uniformly dispersed form in compositions according to the invention on an aqueous basis.

Nonionic, anionic or amphoteric film-forming hair-fixing polymers are especially preferred in the gels according to the invention. Suitable non-ionic polymers include homopolymers or copolymers, which are built up from at least one of the following monomers: vinylpyrrolidone, vinylcaprolactam, vinyl esters such as vinyl acetate, vinyl alcohol, acrylamide, methacrylamide, alkyl- and dialkylacrylamide, alkyl- and dialkylmethacrylamide, alkylacrylate, alkylmethacrylate, propylene glycol or ethylene glycol, wherein the alkyl groups in these monomers preferably have from one to seven carbon atoms, especially preferably from one to three carbon atoms. For example, homopolymers of vinyl caprolactam, of vinyl pyrrolidone or of N-vinylformamide, are especially suitable. Additional suitable synthetic non-ionic film-forming hair-fixing polymers are e.g. copolymerizateS of vinyl pyrrolidone and vinyl acetate, terpolymers of vinyl pyrrolidone, vinyl acetate and vinyl propionate, polyacrylamide, which, for example, are sold under the trade name AKYPOMINE^{®} P 191 of CHEM-Y, Emmerich, Germany, or SEPIGEL^{®} 305 of SEPPIC, USA; polyvinyl alcohols, which, for example, are marketed under the trade name ELVANOLe of DuPont or VINOL^{®} 523/540 of Air Products, U.S.A., and polyethylene glycol/polypropylene glycol copolymers, which are sold e.g. under the trademark UCON^{®} of Union Carbide. Polyvinyl pyrrolidone, polyvinyl caprolactam and their copolymers with at least one additional non-ionic polymer, especially polyvinyl pyrrolidone/vinyl acetate copolymers are especially preferred.

Suitable polymers with acid groups include especially copolymers of acrylic acid or methacrylic acid with monomers selected from the group consisting of acrylic acid or methacrylic acid esters, acryl amides, methacrylamides and vinylpyrrolidones, homopolymers of crotonic acid and copolymers of crotonic acid with monomers selected from the group consisting of vinyl esters, acrylic acid or methacrylic acid esters, acrylamides and methacrylamides. A suitable natural polymer is, for example, shellac.

Preferred polymers with acid groups include cross-linked or uncross-linked vinyl acetate/crotonic acid copolymers. Similarly partially esterified copolymers between vinyl methyl ether and maleic acid anhydride are also preferred. Additional preferred anionic polymers include, e.g., terpolymers of acrylic acid, alkyl acrylate and N-alkylacrylamide, especially acrylic acid/ethyl acrylate/N-t-butylacrylamide terpolymer, terpolymers of vinyl acetate, crotonate and vinyl alkanoate, especially vinyl acetate/crotonate/vinyl neodecanoate copolymers, and copolymers of acrylic acid or methacrylic acid and acrylic acid alkyl esters or methacrylic acid alkyl esters,
wherein the alkyl groups preferably contain from one to seven carbon atoms.

Suitable amphoteric hair-fixing polymers are those polymers, which contain basic or cationic groups as well as acidic or anionic groups as additional functional groups. The basic or cationic groups are, for example, primary, secondary and tertiary amine groups. For example, suitable amphoteric polymers are, for example, copolymers made from alkylacrylamides (especially octylacrylamide), alkylaminoalkylmethaclylates (especially t-butylaminoethylmethacrylate), and two or more monomers, comprising acrylic acid, methacrylic acid or their esters, such as those which are obtainable under the trademarks AMPHOMER^{®} and AMPHOMER^{®} LV-71 of National Starch, U.S.A. Further examples of suitable copolymers include those copolymers of acrylic acid, methacrylate and methacrylamideoPrOPYltrimethylammonium chloride (INCI: poIyquaternium-47), which are sold under the trade name MERQUAT^{®} 2001 of Calgon, Pittsburgh, U.S.A., those copolymers made from acrylamidopropyltrimethyl ammonium chloride and acrylates, such as those sold under the trademark W 37194^{®} by Stockhausen or those copolymers made from acrylamide, acrylamidopropyltrimethylammonium chloride, 2-amidopropylacrylamide sulfonate and dimethylaminopropylamine (DMAPA)(INCI: Polyquaternium-43), such as those marketed under the trademark BOZEQUAT^{®} 4000 of Societe Francaise Hoechst. Suitable polymers made with monomers carrying betaine groups, such as copolymers of methacryloylethylbetaine and two or more monomers made from acrylic acid or their simple esters, known under the INCI designation methacryloyl ethyl betaine/acrylates copolymer.

### Optional Additive Ingredients

The compositions according to the invention can also contain conventional additive ingredients suitable for hair treatment compositions. These additive ingredients include, e.g. moisturizers; perfume oils in an amount of 0.1 to 0.5 percent by weight; bactericidal and fungicidal agents, for example, 2,4,4 trichloro-2-hydroxydiphenyl ether or methyl chloroisothiazolione, in an amount of from 0.01 to 1.0 percent by weight; buffer substances, such as sodium citrate or sodium phosphate, in an amount of from 0.1 to 1.0 percent by weight; dyestuffs, for example, fluorescein sodium salt, in an amount of about 0.1 to 1.0 percent by weight; care materials, such as plant and vegetable extracts, protein and silk hydrolyzates, lanolin derivative compounds, in an amount of 0.1 to 5 percent by weight; light protective materials, antioxidants, radical trapping agents, anti-flaking active ingredients, in an amount of about 0.01 to 2 percent by weight; luster-imparting ingredients, vitamins, softening agents, combability improving agents and de-fatting agents.

If the gel shall impart luster to the hair, the gel may also comprise physiologically compatible silicone derivative compounds, such as volatile or non-volatile silicone oils or high-molecular-weight siloxane polymers in an amount of from 0.05 to 15 percent by weight, preferably from 0.05 to 10 percent by weight. However, if a matte finish shall be achieved, silicone derivate compounds may not be necessary. In fact, for a matte finish it may be desirable not to use any silicone derivate compounds, as these substances typically impart luster to the hair.

### Preparation Process

The compositions according to the invention can be made by a process in which the ingredients are first dissolved in an aqueous solvent. The amount of saccharide, thixotropic gel-forming ingredient and the type and the amount of the additional cosmetic additive ingredients are selected so that a solid, form-stable gel can be formed. In the event that the gel-former or the additional additive ingredients are not completely soluble at room temperature, heat can be used to dissolve those materials, e.g. at about 40 to 80° C. Then the resulting solution is allowed to stand until the solution solidifies to form the gel. Advantageously additional external cooling at least 50 to 55° C. or lower accelerates the solidification process.

### Packaging

Due to the solid, form-stable consistency of the gel of the present invention, it is preferred that the gel is filled into vessels or like containers which allow easy, direct access with the fingers rather than filling the gel into tubes, wherein the product has to be pressed out of the packaging prior to applying it onto the fingers.

### EXAMPLES

The following gel compositions have been prepared

Comparative Example 1 (ratio of saccharide to gel-forming ingredient = 0.5 : 1)

| **Ingredient** | **Wt%** |
|---|---|
| Water, demineralized | 80.8 |
| Lithium Magnesium Sodium Silicate (Laponite XLG) | 12.8 |
| Sucrose (Pure Cane Granulated Sugar) | 6.4 |

Comparative Example 2 (ratio of saccharide to gel-forming ingredient = 1 : 1)

| **Ingredient** | **Wt%** |
|---|---|
| Water, demineralized | 87.2 |
| Lithium Magnesium Sodium Silicate (Laponite XLG) | 6.4 |
| Sucrose (Pure Cane Granulated Sugar) | 6.4 |

Example 3 (ratio of saccharide to gel-forming ingredient = 1.875 : 1)

| **Ingredient** | **Wt%** |
|---|---|
| Water, demineralized Lithium Magnesium Sodium Silicate (Laponite | 81.6 |
| XLG) | 6.4 |
| Sucrose (Pure Cane Granulated Sugar) | 12.0 |

Example 4 (ratio of saccharide to gel-forming ingredient = 3.75 : 1)

| **Ingredient** | **Wt%** |
|---|---|
| Water, demineralized | 69.6 |
| Lithium Magnesium Sodium Silicate (Laponite XLG) | 6.4 |
| Sucrose (Pure Cane Granulated Sugar) | 24.0 |

Example 5 (ratio of saccharide to gel-forming ingredient = 4.75 : 1)

| **Ingredient** | **Wt%** |
|---|---|
| Water, demineralized | 63.2 |
| Lithium Magnesium Sodium Silicate (Laponite XLG) | 6.4 |
| Sucrose (Pure Cane Granulated Sugar) | 30.4 |

Comparative Example 6 (ratio of saccharide to gel-forming ingredient = 6.7 : 1)

| **Ingredient** | **Wt%** |
|---|---|
| Water, demineralized | 50.6 |
| Lithium Magnesium Sodium Silicate (Laponite XLG) | 6.4 |
| Sucrose (Pure Cane Granulated Sugar) | 43.0 |

The Index of Viscosity for each example has been determined by texture analysis, using a TA-XT Plus Texture Analyser available from Stable Micro Systems Ltd, Surrey, UK.

The test is carried out according to the manual "TA-XT*Plus* Application Study", REF: GEL5/BEC, Revised: Jan '00, Copyright 2000 by Stable Micro Systems Ltd.

However, deviating from this instruction, the test is done at 20°C (instead of 25°C) in a climatised room, where the sample is stored over night prior to testing.

The sample container is fastened to prevent it from turning or lifting and the probe is calibrated to a starting distance that is a distance of 30 mm above the sample surface.

Also deviating from the instructions provided in the "TA-XT*Plus* Application Study", the disc proceeds to penetrate to a depth of 20 mm into the sample.

The Index of Viscosity of the gel compositions according to Example 1 to 6 is determined as described in the "TA-XT*Plus* Application Study". The results are as follows:

| **Example No.** | **Index of Viscosity [g s]** |
|---|---|
| 1 | - 4340 |
| 2 | - 166 |
| 3 | - 840 |
| 4 | - 1064 |
| 5 | - 1278 |
| 6 | - 1972 |

The gel composition of (comparative) Example 1 is extremely firm and rather inhomogeneous due to the high amount of gel-forming ingredient and the relatively small amount of saccharide (compared to the amount of gel-forming ingredient). Also, the gel composition has very little hair styling ability due to the low saccharide amount relative to the amount of gel-forming ingredient.

The gel composition of (comparative) Example 2 has a low viscosity and shows the rheology of a typically gel composition usually sold in tubes.

The gel compositions of (comparative) Example 6 are also relatively firm even though the amount of gel-forming ingredient is the same as in Examples 3 and 4 (which are examples according to the present invention). This is believed to be due to the relatively high amount of saccharide (compared to the amount of gel-forming ingredient).

Thus, the viscosity and overall rheology does not only depend on the weight percentage of gel-forming ingredient in the composition but also on the ration of saccharide to gel-forming ingredient.

Examples 3, 4 and 5 are homogeneous, form-stable compositions according to the present invention.

All gel compositions provide a matte (i.e. non-shiny) finish to the hair.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

All documents cited in the Detailed Description of the Invention are, in relevant part, incorporated herein by reference; the citation of any document is not to be construed as an admission that it is prior art with respect to the present invention. To the extend that any meaning or definition of a term in this written document conflicts with any meaning or definition of the term in a document incorporated by reference, the meaning or definition assigned to the term in this written document shall govern.

While particular embodiments of the present invention have been illustrated and described, it would be obvious to those skilled in the art that various other changes and modifications can be made without departing from the spirit and scope of the invention. It is therefore intended to cover in the appended claimed all such changes and modifications that are within the scope of this invention.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A solid, form-stable gel for hair treatment comprising
- at least one saccharide, and
- at least one gel-forming ingredient, and
- an aqueous or aqueous-alcoholic base
wherein the ratio of the at least one saccharide to the at least one gel-forming ingredient is between 1.5 : 1 and 5.5 : 1, and
wherein the gel has an Index of Viscosity at 20°C of between - 500 g s and -1500 g s.

2. Gel according to claim 1, wherein the saccharide is a monosaccharide, or a oligosaccharide consisting of from two to ten monossacharide units.

3. Gel according to claim 1 or 2, wherein the saccharide is a monosaccharide, a dissacharide or a trisaccharide.

4. Gel according to any of the preceding claims, wherein the saccharide is selected from the group consisting of glucose, galactose, fructose, maltose, lactose, saccharose and sucrose.

5. Gel according to any of the preceding claims, wherein the amount of saccharide is from 10 % by weight to 35 % by weight.

6. Gel according to any of the preceding claims, wherein the gel-forming ingredient is a thixotropic gel-forming ingredient.

7. Gel according to any of the preceding claims, wherein the gel additionally comprises an emulgator.

8. Gel according to any of the preceding claims, wherein the content of the aqueous or aqueous-alcoholic base is from 60% to 85% by weight.

9. Gel according to any of the preceding claims, wherein the gel comprises below 0.5% by weight of a film-forming hair-fixing polymer.
